# EUROPEAN PATENT SPECIFICATION

(11) **EP 3 777 936 B1**
(45) Date of publication and mention of the grant of the patent: **22.09.2021**
(21) Application number: 19191551.1
(22) Date of filing: 13.08.2019
(51) Int. Cl.: A61M 5/50, A61M 39/10, A61M 39/16

(54) **NON-REMOVABLE CONNECTION SYSTEM**
NICHT ABNEHMBARES VERBINDUNGSSYSTEM
SYSTÈME DE CONNEXION NON AMOVIBLE

(43) Date of publication of application: 17.02.2021
(73) Proprietor: Gambro Lundia AB, 226 43 Lund (SE)
(72) Inventor: VERNIN, Guillaume, F-69330 MEYZIEU (FR)
(74) Representative: PGA S.p.A., Milano, Succursale di Lugano

(56) References cited:
- EP-A1- 2 620 178
- WO-A1-2016/012834
- GB-A- 2 451 891
- US-A1- 2014 243 797

## Description

### FIELD OF THE INVENTION

The present invention relates to a non removable connection system for medical devices particularly configured to be connected to a fluid line, such as a blood line, an infusion line or a monitoring line. In particular said connection system may be connected to the fluid lines of a dialysis machine or may be directly attached to the dialysis machine or to an accessory of the disposable set/dialysis machine.

The present invention also refers to a disposable comprising fluid lines of a blood treatment apparatus, such as a dialysis machine, and said connection system.

### BACKGROUND

Common connection systems for medical purposes are already known in the state of the art, for example used to connect fluid lines each other or to a medical machine. In blood treatment apparatuses, such as dialysis machines, connectors are commonly used for connecting fluid lines, for example for connecting in a fluid-tight manner return lines and access lines to respective vascular access systems of the patient, and/or for connecting infusion lines or monitoring lines to the blood or fluid circuit. In blood treatment apparatuses, Luer-type connectors are frequently used.

Besides the fact of ensuring the fluid-tight sealing and the suitability to be used in the medical field, connectors are required, in specific cases, to avoid voluntary or involuntary disconnections: for example, where the connector is used on a blood line of a dialysis machine, a disconnection during an operative condition of the machine would lead to dangerous consequences potentially lethal for the patient. Any undesired disconnection may lead to sterility issues and requires the operator intervention, sometimes including the interruption of the treatment.

Document US8882726 is directed to a drug delivery device defining a reservoir for containing a liquid, such as a medicine. The device comprises a distal end presenting an end-piece traversed by a channel along a longitudinal axis. The end-piece is surrounded by a collar extending from the distal end, wherein the collar is provided with a plurality of outer longitudinal ridges running on the whole circumference of the collar: all the longitudinal ridges have each a sloped surface. The device also comprises a tubular wall having an inner duct, wherein an interface of the tubular wall is associable with the end-piece to define a fluid tight connection. The tubular wall is also provided with an outer flange configured to be engaged with a thread arranged in an inner lateral wall of the collar to allow axial engagement between the tubular wall and the end-piece of the device. The tubular wall also comprises a skirt provided with inner longitudinal projections able to cooperate with the longitudinal ridges and their sloped surfaces, to allow a screwing rotation of the tubular wall and to prevent unscrewing rotation of the same.

The applicant points out some drawbacks affecting the device described in US8882726, from manufacturing to ease of use, as well as the impossibility of using it in combination with blood treatment apparatuses. In particular, such connector system would cause twist of the fluid line during connection, determining unwanted restrictions of the fluid line cross section, which might reduce drug or blood flow rate or determine damage of the fluid line itself. Moreover, the architecture of the connector comprises several undercuts and recesses, which makes production very complex and, therefore, expensive.

Document GB2451891A1 discloses a first connector releasably secured to a second connector formed by a collar rotatably mounted over and about an insert. Male and female key elements provide means to avoid disangagement of connectors.

Document US20140243797A1 discloses a male Luer connector including a body having a first end configured to be connected to a fluid line, a second end opposed to the first end, and a body longitudinal axis. The second end is configured to be inserted into and form a Luer slip connection with a female Luer connector. The male Luer connector also includes a locking collar that is connected to the body in a manner such that the locking collar rotates about the longitudinal axis.

Document EP2620178A1 discloses a connector for medical infusion lines, transfusion lines, and the like, comprising a tubular body having at one end a male connector including an inner tubular element and an outer hollow element, which is internally threaded and can be coupled by screwing to a complementary female connector. The line connector further includes an outer manoeuvring sleeve coupled in unidirectional rotation to the body in the direction corresponding to screwing of the male connector with respect to the complementary female connector and free to turn in the opposite direction.

### OBJECT OF THE INVENTION

The object is therefore to at least partially solve one or more of the drawbacks and/or limitations of the previous solutions.

A first object is to provide a non removable connector for medical applications suitable for connecting disposable components, such as fluid lines.

An aim is to avoid the need of multiple interventions and checks during the set-up of a medical apparatus and during the following treatment of a patient.

A further aim is to reduce the risk for the connection to untighten, either due to human error or to other causes, such as mechanical/thermal stress on the connectors.

Another object is to provide a non removable connector easy to produce and cheap.

These objects and more, which will appear more from the following description, are substantially achieved by a connection system and a disposable set in accordance with one or more of the following claims.

The invention is as defined in claim 1.

### DRAWINGS

Some embodiments and some aspects of the invention will be described below with reference to the attached drawings, provided for illustrative purposes only, wherein:
- Figures 1 and 2 are a perspective views of the connection system, disposed in a locked condition, according to the present invention;
- Figures 2A and 2B are a cross section views of the connection system of figure 2;
- Figures 3 is an exploded view of the connection system according to the present invention;
- Figure 3A is a cross section view of figure 3;
- Figure 4 is a perspective view of the connection system, disposed in an unlocked condition, according to the present invention;
- Figure 4A is a cross section view of figure 4;
- Figures 5 and 6 are different embodiments of a disposable set according to the present invention.

### DEFINITIONS

In this detailed description, corresponding parts illustrated in the various figures are indicated with the same numerical references. The figures may illustrate the invention by means of non-scale representations; therefore, parts and components illustrated in the figures relating to the object of the invention may relate exclusively to schematic representations.

### Upstream and/ downstream

The terms upstream and downstream refer to a direction or trajectory of advancement of a fluid configured to flow within the connector or along the fluid line during normal usage of the apparatus.

### Non-Removable

By the wording 'non-removable' is meant that the connection system is in a locked condition under normal use. As below explained, in the locked condition of the connection system at least one locking projection 24 of the collar 20 and at least one locking projection 14 of the second connector 11 are in a coupled configuration, and a hooking portion 23 of the collar 20 and a hooking portion 13 of the second connector 11 are in an engaged configuration; in said locked condition the collar 20 is irremovably engaged to the second connector 11 through an engagement rotation and the locking projection 14 and the hooking portion 13 of the second connector 11 respectively cooperating with the locking projection 24 and the hooking portion 23 of the collar 20 prevent disengagement of the first connector 1 from the second connector 11.

### DETAILED DESCRIPTION

### Connection system 100

Reference number 100 is directed to a connection system, as shown in figures from 1 to 4, for a medical apparatus: the connection system 100 may be used to connect, in a fluid tight manner, fluid lines 51 of a medical machine 200, such as a blood treatment apparatus, in particular a dialysis machine, wherein the fluid lines 51 may be a blood circuit 50a of a disposable set 50, an infusion line 54, a monitoring line 55, an access line 52 or a return line 53.

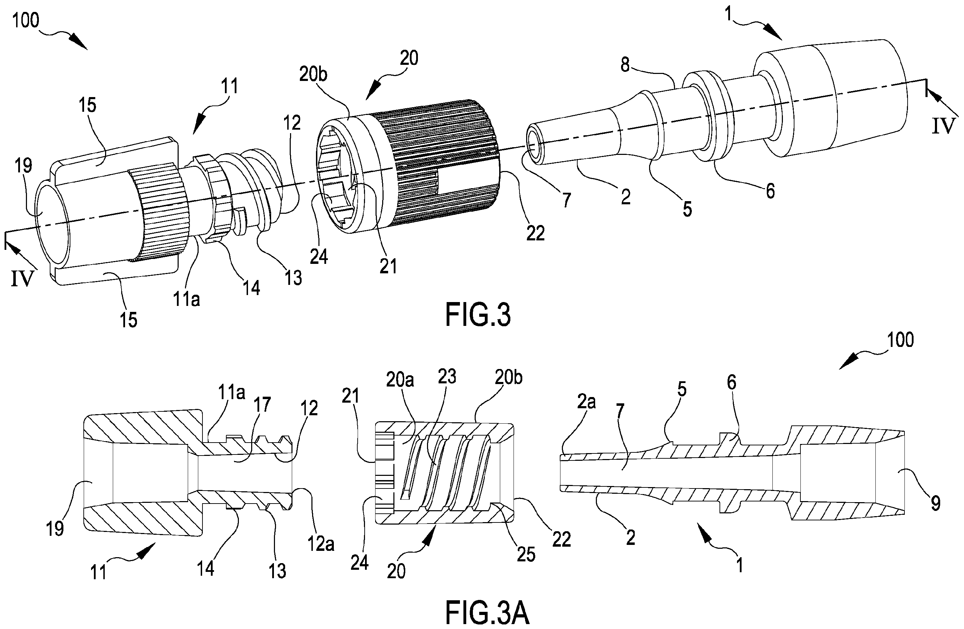

The connection system 100 comprises a first connector 1, shown in the exploded view of figure 3 and in the respective cross section in figure 3A, comprising a main body having tubular shape and defining an internal channel 7 for fluid passage: the main body of the first connector 1 and the internal channel 7 preferably extend in length along the same fluid flow axis, between a connection port 9 configured to be fixed/connected in a fluid tight manner to a fluid line 51 or directly to the medical machine 200 (or an accessory), and a coupling portion 2. The connection port 9 may be a male or female connection port. Usually, a tube end portion is irremovably fixed inside the connection port via e.g., gluing or bonding. The coupling portion 2 of the first connector 1 has preferably tubular shape and includes a tip portion 2a surrounding the internal channel: the coupling portion 2 has a tapered external surface, so that a diameter of the coupling portion 2 reduces towards the tip portion 2a of the coupling portion 2. In particular, the tapered external surface of the tip portion 2a of the coupling portion 2 has a taper angle comprised between 1° and 6°, more in particular comprised between 3° and 4°, more in detail the taper angle is equal to 3.44°. In an embodiment, the first connector 1, and in particular the coupling portion 2 of the first connector 1, is a Luer type connector: this means that dimensions of the coupling portion 2, the diameter of the internal channel 7 and the overall length of the first connector 1 are consistent with the Luer standards. In a non-limiting embodiment, the first connector 1 has a length comprised between 2 cm and 4 cm, and the internal channel 7 has an average diameter comprises between 2 mm and 3 mm.

The first connector 1 further comprises a main abutment portion 5 arranged on an external lateral wall of the main body, wherein the main abutment portion 5 defines a protrusion radially extending outward from the main body. The abutment portion 5 may have circular shape, for example a ring coupled to the main body extending outwardly.

In an embodiment, the first connector 1 may further comprise an auxiliary abutment portion 6 arranged on the external lateral wall of the main body, wherein the auxiliary abutment portion 6 is axially distal from the main abutment portion 5 by an axial gap, so that a recess 8 is defined between the main abutment portion 5 and the auxiliary abutment portion 6. As shown in figures 3 and 3A, the auxiliary abutment portion 6 is axially interposed between the connection port 9 and the main abutment portion 5: similarly, the main abutment portion 5 is interposed between the coupling portion 2 and the auxiliary abutment portion 6.

In the shown embodiment, the first connector 1 is a male connector and it is made in one single piece preferably in plastic material, such as PVC, MBS, PC, or in metallic material, such as stainless steel.

The connection system 100 further comprises a second connector 11, shown in the exploded view of figure 3 or in the cross section view of figure 3A, presenting a main body having tubular shape and defining an internal channel 17 for fluid passage: the main body of the second connector 11 and the internal channel 17 preferably extend in length, along the same fluid flow axis, between a connection port 19 configured to be fixed/connected in a fluid tight manner to a fluid line 51 or directly to the medical machine 200 (or an accessory), and a coupling portion 12. The connection port 19 may be a male orfemale connection port. Usually, a tube end portion is irremovably fixed inside the connection port 19 via e.g., gluing or bonding. The coupling portion 12 of the second connector 11 is configured to be connected to the coupling portion 2 of the first connector 1 in a fluid tight manner, so that a fluid is able to flow between the first and the second connectors 1, 11, and vice versa, in particular wherein the fluid is able to flow between the connection port 9 of the first connector 1 and the connection port 19 of the second connector 11, and vice versa. When the coupling portion 2 of the first connector 1 is connected to the coupling portion 12 of the second connector 11, the fluid flow axis of the first connector 1 coincides with the fluid flow axis of the second connector 11, and the internal channels 7, 17 respectively of the first and the second connectors 1, 11 define a unique internal channel for fluid passage extending from the connection port 9 of the first connector 1 to the connection port 19 of the second connector 11.

The coupling portion 12 of the second connector 11 has preferably tubular shape and includes a terminal portion 12a surrounding the internal channel 17 and having a tapered internal surface, so that a diameter of the tapered internal surface increases towards the terminal portion 12a. According to the disclosed embodiment, the tapered internal surface of the terminal portion 12a of the second connector 11 is counter shaped to the tapered external surface of the tip portion 2a of the first connector 1. In other words, both the coupling portion 2 of the first connector 1 and the coupling portion 12 of the second connector 11 have conical shape.

The tip portion 2a of the first connector 1 is configured to enter into and to contact the terminal portion 12a of the second connector 11, to define a fluid tight connection: the tapered external surface is configured to couple with the tapered internal surface to connect the first and the second connectors 1, 11 in a fluid tight manner. In particular, the tapered internal surface of the terminal portion 12a of the coupling portion 2 has a taper angle comprised between 1° and 6°, more in particular comprised between 3° and 4°, more in detail the taper angle is equal to 3.44°. In the specific embodiment, the taper angle of the coupling portion 2 of the first connector 1 is equal to the taper angle of the coupling portion 12 of the second connector 11. In an embodiment, the second connector 11, and in particular the coupling portion 12 of the second connector 11, is a Luer type connector: this means that dimensions of the coupling portion 12, the diameter of the internal channel 17 and the overall length of the second connector 11 are consistent with the Luer standards. In an embodiment, the second connector 11 has a length comprised between 2 cm and 3 cm, and the internal channel 17 has an average diameter comprises between 3 mm and 4 mm.

The second connector 11 further comprises a hooking portion 13 arranged on an external lateral wall 11a of the main body of the second connector 11: in particular the hooking portion 13 may comprise a thread, preferably arranged in the proximity of the coupling portion 12 of the second connector 11. The threads of the hooking portion 13 preferably extend from the terminal end 12a of the second connector 11 and preferably emerge from the external lateral wall 11a of the main body of the second connector 11. In an alternative embodiment, the hooking portion 13 of the second connector 11 may comprise a respective undercut joint (embodiment not shown in the attached figures).

The second connector 11 further comprises at least one locking projection 14 arranged on the same external lateral wall 11a of the main body of the second connector 11. In an embodiment, shown in figure 3, the at least one locking projection 14 is interposed between the hooking portion 13 and the connection port 19: similarly, the hooking portion 13 is interposed between the at least one locking projection 14 and the terminal end 12a of the second connector 11. The at least one locking projection 14 radially emerges outwardly from the main body of the second connector 11: in particular the at least one locking projection 14 comprises a plurality of socket teeth, wherein the socket teeth are wedge-shaped or have a saw tooth shape. The socket teeth are preferably arranged all around the tubular main body of the second connector 11, as shown in figure 3.

In the embodiment, the second connector 11 is a female connector and it is made in one single piece preferably in plastic material, such as PVC, MBS, PC or in metallic material, such as stainless steel.

The second connector 11 may also comprise gripping means arranged on the external lateral wall 11a at the connection port 19: the gripping means are configured to allow an operator to grab firmly the second connector 11 especially when connection with the first connector 1 is required. The gripping means 15 preferably comprise tabs, in particular two tabs diametrically opposed, emerging radially outwardly from the main body of the second connector 11.

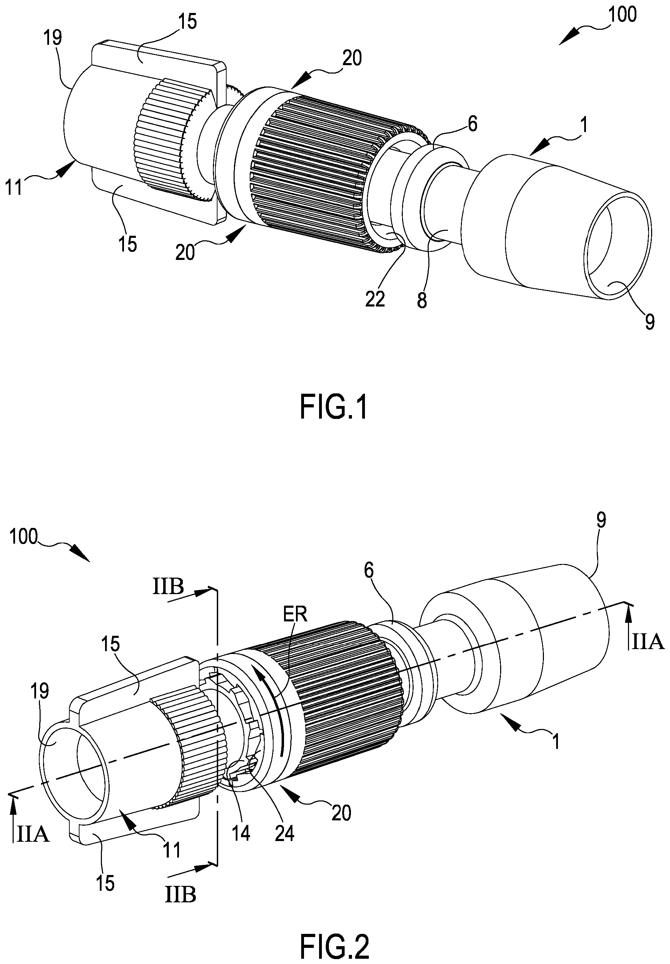

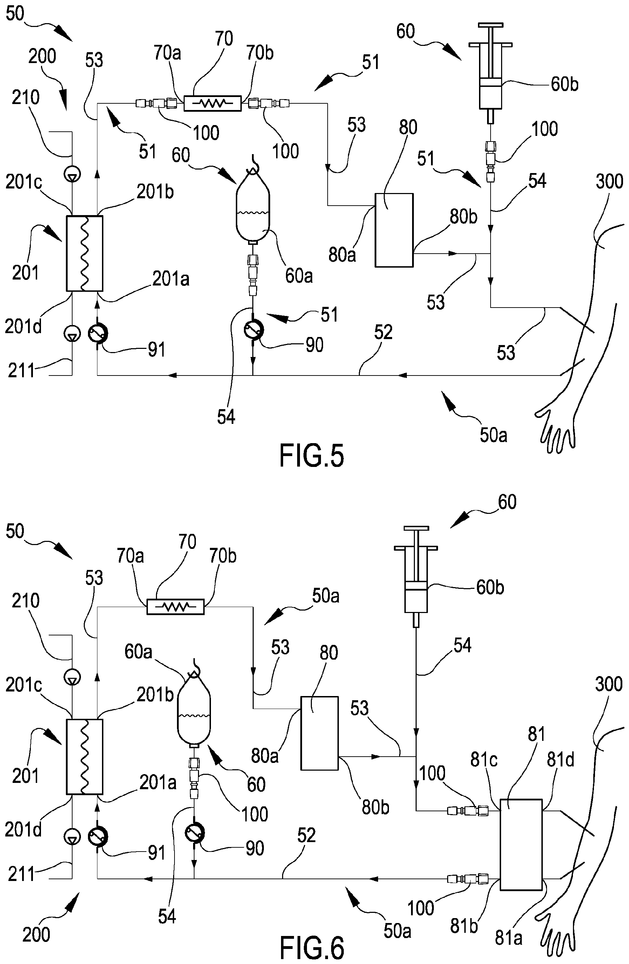

The connection system 100 further comprises a collar 20, shown in the exploded view of figure 3 and in the cross section view of figure 3A, presenting a main body having tubular shape: in particular the collar extends in length between a first opening 21 and a second opening 22, and the tubular main body comprises an external lateral wall 20b and an internal lateral wall 20a. In an embodiment not shown in the attached figures, the main body of the collar 20 may have polygonal shape, such as squared, rectangular or a combination of polygonal and tubular shapes. The collar 20 comprises an internal channel laterally limited by the first and the second openings 21, 22, wherein the opening 21 is configured to receive through insertion the second connector 11, in particular to receive the coupling portion 12, the hooking portion 13 and the at least one locking projection 14 of the second connector 11, while the second opening 22 allows the collar 20 to be mounted over, and coupled to, the first connector 1, as shown in figure 7. In more detail, the second opening 22 receives in insertion the first connector 1, so that the collar surrounds externally at least part of the first connector 1 and the main body of the collar 20 is coaxial with the first connector 1. In particular the collar 20 comprises, preferably at the second opening 22, a respective abutment portion 25 configured to contact the main abutment portion 5 of the first connector 1 when disposed in an abutment configuration, so that the collar 20 is axially constrained with respect to the first connector 1 at least along one axial direction. In particular the collar 20 is axially constrained towards the coupling portion 2 of the first connector 1, so that the collar 20 is prevented to axially move towards the coupling portion 2 of the first connector 1 beyond a certain limit. The abutment portion 25 of the collar 20, as shown in the section of figure 6, is arranged at the second opening 22 of the collar 20 and extends radially inwardly. In the disclosed embodiment shown in figures 1 to 4, the first connector comprises both the main and the auxiliary abutment portions 5, 6, so that the abutment portion 25 of the collar is interposed between the main abutment portion 5 and the auxiliary abutment portion 6 of the first connector: therefore, the collar is a axially constrained to the first connector 1 through the main and auxiliary abutment portions 5, 6 of the first connector 1, so that an axial movement of the collar 20 with respect to the first connector 1 is substantially prevented. Alternatively or in addition, the collar 20 may be axially movable with respect to the first connector 1 within the axial gap defined between said main and auxiliary abutment portions 5, 6 of the first connector 1, and axially constrained to the first connector 1 out of this axial gap. In particular, the collar 20 is axially movable with respect to the first connector 1 of an amount proportional to the distance between the main and the auxiliary abutment portions 5, 6 of the first connector 1. The axial movement is defined along the fluid flow axis of the first connector 1 and/or of the second connector 11. Anyhow, if the first connector comprises the main and the auxiliary abutment portions 5, 6, the abutment portion 25 of the collar 20 is arranged at, and in particular surrounds, the recess 8 of the first connector 1. In this specific embodiment, the second opening 22 has an internal diameter lower than an external diameter of the main abutment portion 5 of the first connector 1, preventing in this way the axial movement.

In other words, the first connector 1 and the collar, although they are distinct bodies both made in a single piece, are axially coupled each other, being the collar 20 movable by rotation with respect to the first connector 1.

In an embodiment, the collar 20 has a length, measured along an axis of its tubular main body, comprised between 8 mm and 25 mm, and a diameter comprised between 7 mm and 15 mm

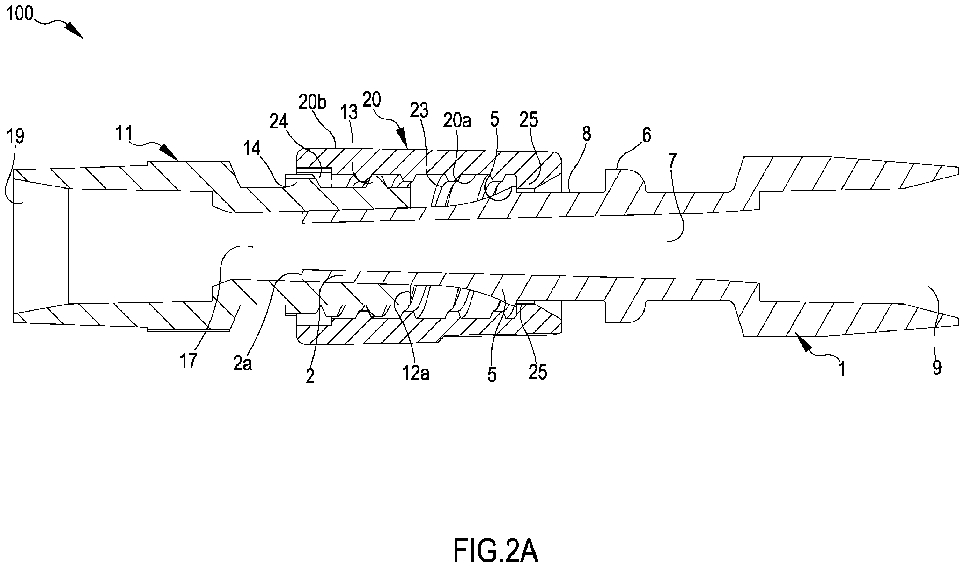

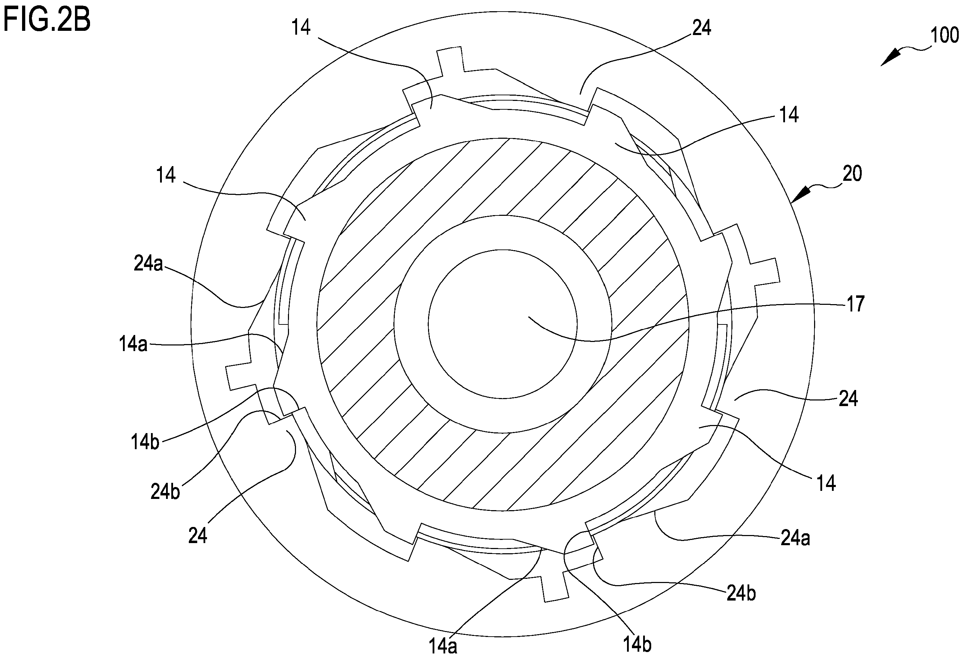

The collar 20 further comprises a respective hooking portion 23 configured to be engaged with the hooking portion 13 of the second connector 11 through an engagement rotation ER along an engagement direction (see figure 2) defining an engaged configuration shown in figures 1 and 2, so that when the connection system 100 is disposed in this engaged configuration the hooking portion 23 of the collar 20 and the hooking portion 13 of the second connector 11 prevent axial removal of the collar 20 from the second connector 11. In an embodiment, the hooking portion 23 of the collar comprises a thread configured to engage the thread of the second connector 11 through this engagement rotation. In an embodiment, the hooking portion 23 is arranged on the internal lateral wall 20a of the collar 20. In other words, in this engaged configuration, the collar 20 is screwed over the second connector 11 through the respective threads, defining an axial constraint between the collar 20 and the second connector 11.

In an alternative embodiment, the hooking portion 23 of the collar 20 comprises an undercut joint (not shown in the attached figures) and the hooking portion 13 of the second connector 11 comprises the respective undercut joint, wherein the undercut joint of the collar 20 is configured to axially engage the undercut joint of the second connector 11 defining a bayonet coupling for example through the same engagement rotation ER, so that an axial movement, in particular an axial separation, of the first and second connectors 1, 11 is prevented: this axial movement or axial separation is considered along the fluid flow axis of the first or second connectors 1, 11. In this alternative embodiment, the engagement rotation is comprised between 10° and 180°, in particular comprised between 15° and 95°. Since the collar 20 is axially coupled to the first connector 1, when the connection system 100 is disposed in this engaged configuration the first connector 1 is axially coupled to the second connector 11, so that disconnection of the first and second connectors 1, 11 is prevented. In particular, at an end stage of the engagement rotation, the hooking portions 13, 23 respectively of the second connector 11 and of the collar 20 contribute to tighten the connection and to prevent disconnection between the coupling portion 2 of the first connector 1 and the coupling portion 12 of the second connector 11. During an engagement rotation ER wherein the collar20 rotates to determine the engagement rotation, the coupling portion 2 of the first connector 1 is allowed to stay aligned with the coupling portion 12 of the second connector 11 due to the fact that the collar 20 is freely movable by rotation with respect to the first connector 1: once the connection between the coupling portion 2 of the first connector 1 and the coupling portion 12 of the second connector 11 is established, the collar 20 is still freely movable by rotation with respect to the first and second connectors 1, 11, so that the subsequent engagement rotation ER does not determined friction between the coupling portion 2 of the first connector 1 and the coupling portion 12 of the second connector 11. In other words and according to an embodiment, during the engagement rotation the first and the second connectors 1, 11 may be aligned each other without reciprocal rotation, while the collar 20 rotates with respect both the first and second connectors 1, 11 to determine engagement of the hooking portions 13, 23.

The collar 20 further comprises a respective at least one locking projection 24 engageable to the at least one locking projection 14 of the second connector 11 through the engagement rotation ER of the collar 20 with respect to the second connector 11 along the engagement direction. The connection system 100 may be disposed in a coupled configuration, wherein the at least one locking projection 24 of the collar 20 cooperates with the at least one locking projection 14 of the second connector 11, preventing a disengagement rotation of the collar 20 with respect to the second connector 11: this disengagement rotation is a rotation in a opposite direction with respect to the engagement rotation. The coupled configuration is defined at least at an end stage of the engagement rotation between the collar 20 and the second connector 11, meaning that when the engagement rotation is complete, a disengagement rotation of the collar 20 with respect to the first connector 1 is prevented. In addition, the coupled configuration may be defined at an intermediate or initial stage of the engagement rotation ER: this means that, as soon as the hooking portion 23 of the collar 20 engages the hooking portion 13 of the second connector 11, the locking projections 14, 24, respectively of the second connector 11 and of the collar 20, also cooperate each other, preventing the disengagement rotation.

In an embodiment, this locking projections 24 are arranged on the internal lateral wall 20a of the collar, so that the locking projection 24 and the hooking portion 23 of the collar 20 are arranged side by side. In particular, the at least one locking projection 24 of the collar may comprise a plurality of socket teeth, wherein the socket teeth are wedge-shaped or have a saw tooth shape. The at least one locking projection 14 radially emerges inwardly from the internal lateral wall 20a of the collar, as shown in figure 3. When the socket teeth of the collar 20 face, and in particular contact, the socket teeth of the second connector 11, the engagement rotation is allowed and the disengagement rotation is prevented.

The socket teeth of both the second connector 11 and the collar 20 are arranged circumferentially all around the external lateral wall 11a of the second connector 11 and circumferentially all around the internal lateral wall 20a of the collar 20. The socket teeth radially define recesses and protrusions alternately, so that the second connector 11 defines a maximum external diameter of encumbrance measured at the protrusions of the socket teeth, and wherein the collar 20 defines a minimum internal diameter measured at the protrusions of the socket teeth of the collar 20: this maximum external diameter of encumbrance is higher than this minimum internal diameter so that, when the socket teeth of the second connector 11 are faced to or in contact with the socket teeth of the collar 20, the protrusions of the socket teeth of the second connector 11 are placed into the recesses of the socket teeth of the collar 20 and vice versa. This is clearly shown in the cross section view of figure 2B, wherein the saw tooth shape of the locking projections allows the engagement rotation and prevents the disengagement rotation.

In an embodiment, the socket teeth of the second connector 11 and of the collar 20 comprise each a respective slide surface 14a, 24a (figure 2B) tilted with respect to the external surface of the second connector 11 and to the internal lateral wall 20a of the collar 20: preferably this slide surface 24a is tilted by an angle lower than 60°. Each of the socket teeth of the second connector 11 and of the collar 20 further comprise respective lock surfaces 14b, 24b which are substantially perpendicular with respect to the external surface of the second connector 11 and to the internal lateral wall 20a of the collar 20: the slide surface 14a, 24a and the lock surface 14b, 24b of each socket tooth are connected together at a vertex defining the protrusion. In other words, the slide surface 14a, 24a and the lock surface 14b, 24b, of one socket tooth define a triangle shaped tooth, wherein these teeth are radially arranged over the internal lateral wall 20a of the collar 20 and over the external lateral wall 11a of the second connector 11. The socket teeth of the collar 20 are oriented in an opposite direction with respect to the socket teeth of the second connector 11, so that the lock surfaces 14b of the socket teeth of the second connector 11 may be faced to the lock surfaces 24b of the collar 20.

Each slide surface 14a of a socket tooth of the second connector 11 is configured to cooperate or contact with a respective slide surface 24a of a socket tooth of the collar 20, so that the engagement rotation is allowed: on the other hand, each lock surface 14b of a socket tooth of the second connector 11 is configured to contact, when the connection system 100 is disposed in the coupled configuration, with a respective lock surface 24b of a socket tooth of the collar 20, preventing therefore the disengagement rotation: in particular, when the connection system 100 is disposed in the coupled configuration, each lock surface 14b of a socket tooth of the second connector 11 abuts against a respective lock surface 24b of a socket tooth of the collar 20.

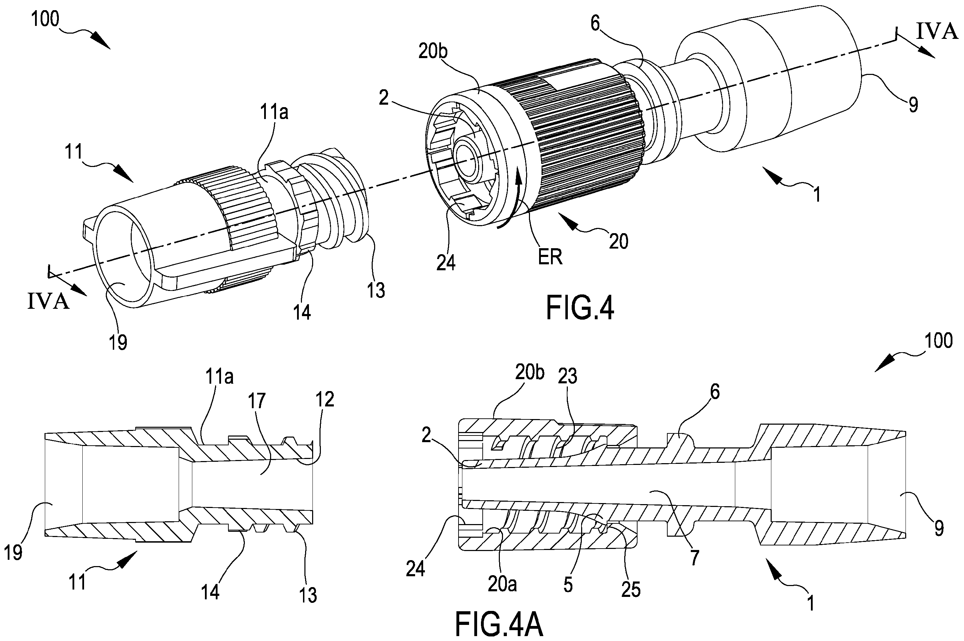

In an embodiment shown in figures 3, 3A, 4 and 4A, the hooking portion 13 of the second connector 11 is interposed between the at least one locking projection 14 and the terminal portion 12a of the coupling portion 12, and the at least one locking projection 24 of the collar 20 is arranged at an inlet of the first opening 21 so that the hooking portion 23 of the collar 20 is interposed between the at least one locking projection 24 and the second opening 22. In an alternative embodiment not shown in the attached figures, the at least one locking projection 14 of the second connector 11 is interposed between the hooking portion 13 and the terminal portion 12a of the coupling portion 12, and the hooking portion 23 of the collar 20 is arranged at the inlet of the first opening 21 so that the at least one locking projection 24 of the collar 20 is interposed between the hooking portion 23 and the second opening 22 of the collar 20.

The connection system 100 is configurable in an unlocked condition, wherein the collar 20 is movable by rotation with respect to the first connector 1 and the at least one locking projection 24 of the collar 20 and the at least one locking projection 14 of the second connector 11 are in an uncoupled configuration wherein the locking projections do not cooperate each other. The unlocked condition is shown in figure 4, wherein the first and the second connectors 1, 11 are separated: when the first and the second connectors 1, 11 are separated, the collar 20 is anyhow axially constrained to the first connector through the main and auxiliary abutment portions 5, 6 of the first connector 1 cooperating with the abutment portion 25 of the collar.

In addition, the unlocked condition may also be defined when the hooking portion 13 of the second connector 11 is engaged with the hooking portion 23 of the collar 20, which defines the engaged configuration: this means that, when an engagement rotation is at an initial or intermediate stage, the at least one locking projection 24 of the collar 20 and the at least one locking projection 14 of the second connector 11 do not cooperate yet, allowing disengagement rotation of the collar 20 with respect to the first connector 1.

The connection system 100 is further configurable in a locked condition, shown in figures 1, 2, and in the section view of figure 2A, wherein the at least one locking projection 24 of the collar 20 and the at least one locking projection 14 of the second connector 11 are in the coupled configuration, and the hooking portion 23 of the collar 20 and the hooking portion 13 of the second connector 11 are in the engaged configuration. In this locked condition, the collar 20 is irremovably engaged to the second connector 11 through said engagement rotation: in particular, the at least one locking projection 14 and the hooking portion 13 of the second connector 11 respectively cooperate with the at least one locking projection 24 and the hooking portion 23 of the collar 20 preventing disengagement of the first connector 1 from the second connector 11. Moreover, when the connection system 100 is disposed in the locked condition, the coupling portion 2 of the first connector 1 is connected to and/or inserted into the coupling portion 12 of the second connector 11, so that fluid tightness between the first and the second connector 1, 11 is established. Moreover, in this locked condition, the abutment portion 25 of the collar 20 abuts against the main abutment portion 5 of the first connector 1, preventing the axial movement, and therefore separation, of the first connector with respect to the collar.

In an embodiment, the collar 20, when engaged to the second connector 11 by the respective threads, may present a slight axial movement with respect to the second connector 11, especially at an initial or intermediate stage of the engagement rotation: this may happen if the width of the thread wire of the second connector 11, is lower than the thread pitch of the collar 20, defining a gap in between, which leads to an axial movement of the collar with respect to the second connector. In this case, the locking portions 14 and 24 respectively of the second connector 11 and of the collar 20 should have a width, measured parallel to the fluid flow axis, higher than this gap, in order to prevent decoupling. In the shown embodiment, when the connection system 100 is in the locked condition and/or in the engaged configuration, the fluid flow axis of the first connector 1 coincides with the fluid flow axis of the second connector 11, and the collar is coaxial with this fluid flow axis. According to the above description, it is to be understood that the invention is not supposed to be limited to the disclosed embodiments, but on the contrary, is intended to cover various modifications and equivalent arrangements included within the spirit and the scope of the appended claims.

### Disposable set 50

The invention also refers to a disposable set 50, shown in figures 5 and 6, for a blood treatment apparatus 200, such as a dialysis machine. The disposable set 50 comprises a plurality of fluid lines 51, such as a blood circuit 50a which includes an access line 52 configured to be connected to a patient 300 and to an inlet port 201a of a blood treatment unit 201, such as a hemofilter, an ultrafilter, a hemodiafilter, a dialyzer, a plasmafilter and the like. The blood circuit 50a also comprises a return line 53 configured to be connected to the patient 300 and to an outlet port 201b of the same blood treatment unit 201. In this configuration, blood is supposed to flow from the patient along the access line 52, through the blood treatment unit 201, and towards the return line 53, as shown by the arrows depicted on the fluid lines of figures 5 and 6.

The disposable set 50 may also comprise one or more infusion lines 54 connected to the blood circuit 52 and/or configured to be connected to the patient 300. The infusion line 54 may comprise a container 60 connected to one end of the infusion line and wherein the other end of the infusion line 54 is connected to the blood circuit or to the patient. This container 60 is configured for housing an infusion fluid and is made, for example, of plastic material for medical applications: in particular the container 60 may be a citrate solution container 60a containing a citrate concentrated solution, or calcium solution container 60b containing a calcium concentrated solution, or a substitute fluid solution to be injected into the blood circuit during treatment.

Figures 5 and 6 shows a (pre-) infusion line 54 arranged upstream with respect to the blood treatment unit 201 and connected to a citrate solution container 60a. The infusion line 54 is also placed upstream a blood pump 91 to provide regional anticoagulation to the extracorporeal blood. At least one pump 90 may be associated to the infusion line 54 and configured to determine fluid flow from the container 60 towards the other end of the infusion line 54, for example towards the access line 52 or to the return line 53. In more detail, the pumps 90 and 91 are peristaltic pumps.

Other infusion lines may be included in the circuit and are not represented. For example a pre-infusion line injecting substitution fluid upstream the blood treatment unit 201 and downstream the blood pump 91 may be included, as well as a post-infusion line injecting a substitute solution into the return line 53, for example into the venous bubble trap (not shown).

Figures 5 and 6 also show an additional infusion line 54 arranged downstream with respect to the to the blood treatment unit 201 and connected to the calcium solution container 60a, for example a syringe configured for housing a calcium solution. This infusion line is intended to reestablish the ion balance in a regional anticoagulation procedure.

The blood pump 91 is associated to the access line 52 and/or to the return line 53 to determine blood flow through the treatment unit 201.

The disposable set 50 may also comprise a monitoring line 55, not shown in the attached figures, connected to the blood circuit 50a or to any of the infusion lines 54, and configured e.g., to be connected to a pressure or to temperature gauge for parameter detection or to be used as a site access for blood sampling.

The disposable set 50 may also comprise a warmer accessory 70, such as a bag or a line and schematically shown in figures 5 and 6, connected to the blood circuit 50a (e.g. downstream the treatment unit 201) and configured to warm up the blood before return to the patient 300. In particular the warmer accessory 70 is connected in series to the return line 53, so that an inlet 70a of the warmer accessory 70 is connected to an upstream tract of the return line 53, and an outlet 70b of the warmer accessory 70 is connected to a downstream tract of the return line 53.

The disposable set 50 further comprises at least one connection system 100 as previously described, which may be connected to the access line 52, the return line 53, to the infusion line 54 and the monitoring line 55. In figure 5 the connection system 100 connects the (calcium) infusion line 54 to the blood circuit; alternatively or in addition the connection system may be used to connect the citrate solution container 60a to the pre blood pump infusion line 54. Moreover, the connections system 100 may connect the inlet 70a and the outlet 70b of the warmer accessory 70 to the return line 53, in order to prevent any undesired disconnection.

The disposable set 50 also comprises the blood treatment unit 201 having a blood inlet 201a connected to the access line 52, a blood outlet 201b connected to the return line 53, a fluid inlet 201c connected to a fluid delivery line 210 configured to be connected to a source of fresh treatment fluid (not shown in the attached figures), and a fluid outlet 201d connected to a fluid output line 211 configured to be attached to an exhaust unit, such as a discharge bag. In an embodiment not shown in the attached figures, the connection system 100 may also be used to connected the blood inlet 201a of the blood treatment unit 201 to the access line 52, the blood outlet 201b to the return line 53, the fluid inlet 201c to the fluid delivery line 210 and the fluid outlet 201 d to the fluid output line 211 (if proper or necessary). The disposable set 50 may also comprise a disposable accessory 80, such as a gas exchanger, an oxygenator or an auxiliary fluid arrangement, connected to the blood line 50a, in particular a gas exchanger 80 may be connected in series to the return line 53. The gas exchanger 80 is configured to remove CO₂ from the blood before returning it to the patient 300. The gas exchanger comprises a blood inlet 80a and a blood outlet 80b for connection to the return line 53: the connection system 100, in an embodiment not shown in the attached figures, may further be used for connecting the blood inlet 80a of the gas exchanger 80 to an upstream tract of the return line 53, and the blood outlet 80b to a downstream tract of the return line 53.

The disposable set may also comprise an add-on accessory 81 connected to the blood circuit 50a through the connection system 100 as shown in figure 6. The add-on accessory 81 is interposed between the patient (in particular the patient vascular access) and the access and return lines of the blood circuit; the add-on accessory 81 comprises an arterial blood inlet 81a configured to be connected to an arterial access of the patient 300, an arterial blood outlet 81b connected to the access line 52 of the disposable set 50, a venous blood inlet 81c connected to the return line 53 of the disposable set 50, and a venous blood outlet 81d configured to be connected to a venous access of the patient 300. The connection system may be interposed in connection between the arterial blood outlet 81b and the access line 52, and between the venous blood inlet 81c and the return line 53.

Obviously, the disposable set may include several additional elements not represented/described since well-known to the skilled person, such as deaeration chambers, pressure monitoring components, infusion or sampling sites, bubble detectors, venous and arterial clamps to stop blood circulation, etc....

The disposable set may be a set for acute or chronic treatments and may assume different configurations with respect to the shown exemplary set.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the scope of the appended claims.

**Parts list**

| | |
|---|---|
| first connector 1 | container 60 |
| coupling portion 2 | citrate solution container 60a |
| tip portion 2a | calcium solution container 60b |
| main abutment portion 5 | warmer accessory 70 |
| auxiliary abutment portion 6 | inlet 70a |
| internal channel 7 | outlet 70b |
| recess 8 | auxiliary disposable accessory 80 |
| connection port 9 | blood inlet 80a |
| second connector 11 | blood outlet 80b |
| external lateral wall 11a | add-on accessory 81 |
| coupling portion 12 | arterial blood inlet 81a |
| terminal portion 12a | arterial blood outlet 81b |
| hooking portion 13 | venous blood inlet 81c |
| locking projection 14 | venous blood outlet 81d |
| internal channel 17 | pump 90 |
| connection port 19 | blood pump 91 |
| collar 20 | non-removable connection system 100 |
| internal lateral wall 20a | medical machine 200 |
| external lateral wall 20a | blood treatment unit 201 |
| first opening 21 | blood inlet 201a |
| second opening 22 | blood outlet 201b |
| hooking portion 23 | fluid inlet 201c |
| locking projection 24 | fluid outlet 201d |
| abutment portion 25 | fluid delivery line 210 |
| disposable set 50 | fluid output line 211 |
| blood circuit 50a | patient 300 |
| fluid line 51 | |
| access line 52 | **List of other references** |
| return line 53 | engagement direction ER |
| infusion line 54 | |
| monitoring line 55 | |

## Claims

1. Non-removable connection system (100) for a medical apparatus, said connection system (100) comprising:
- a first connector (1) comprising a fluid coupling portion (2);
- a second connector (11) comprising a respective fluid coupling portion (12) connectable, in a fluid tight manner, to the fluid coupling portion (2) of the first connector (1), so that a fluid is able to internally flow between the first and the second connector (1, 11), the second connector (11) further comprising a hooking portion (13) and at least one locking projection (14);
- a collar (20) coupled to the first connector (1) and comprising a respective hooking portion (23) engageable to the hooking portion (13) of the second connector (11) through an engagement rotation along an engagement direction (ER), in a engaged configuration the hooking portion (23) of the collar (20) and the hooking portion (13) of the second connector (11) preventing axial removal of the collar (20) from the second connector (11),
wherein the hooking portion (23) of the collar (20) and the hooking portion (13) of the second connector both comprise:
respective threads configured to engage each other through the engagement rotation to define the engaged configuration; or
respective undercut joints configured to engage each other as a bayonet coupling through the engagement rotation to define the engaged configuration;
and wherein the first connector (1) comprises a main abutment portion (5) and the collar (20) comprises a respective abutment portion (25) configured to contact the main abutment portion (5) of the first connector (1) when disposed in an abutment configuration, wherein, in said abutment configuration, the collar (20) is axially constrained with respect to the first connector (1) at least along one axial direction, so that, at least when the connection system (100) is disposed in the engaged configuration, the collar (20) axially engages the first connector (1) to the second connector (11),
wherein the collar (20) further comprises a respective at least one locking projection (24) engageable to the at least one locking projection (14) of the second connector (11) through the engagement rotation of the collar (20) with respect to the second connector (11) along the engagement direction (ER);
wherein by means of the engagement rotation, the connection system (100) is configurable in at least an unlocked condition, wherein the collar (20) is movable by rotation with respect to the first connector (1) and the at least one locking projection (24) of the collar (20) and the at least one locking projection (14) of the second connector (11) are in an uncoupled configuration;
**characterized by** the fact that, in a coupled configuration, the at least one locking projection (24) of the collar (20) and the at least one locking projection (14) of the second connector (11) prevent a disengagement rotation of the collar (20) with respect to the second connector (11), said disengagement rotation being a rotation in a opposite direction with respect to said engagement rotation,
the connection system (100) being configurable from the unlocked condition to a locked condition, wherein the at least one locking projection (24) of the collar (20) and the at least one locking projection (14) of the second connector (11) are in the coupled configuration, and the hooking portion (23) of the collar (20) and the hooking portion (13) of the second connector (11) are in the engaged configuration, in said locked condition the collar (20) being irremovably engaged to the second connector (11) through said engagement rotation and the at least one locking projection (14) and the hooking portion (13) of the second connector (11) respectively cooperating with the at least one locking projection (24) and the hooking portion (23) of the collar (20) preventing disengagement of the first connector (1) from the second connector (11).

2. The connection system of claim 1, wherein:
the first connector (1), in particular the coupling portion (2) of the first connector (1), extends in length along a fluid flow axis;
the second connector (11), in particular the coupling portion (12) of the second connector (11), extends in length along a respective fluid flow axis;
the fluid flow axis of the first connector (1) being aligned, at least when the coupling portion (2) of the first connector (1) is connected to the coupling portion (12) of the second connector (11), to the fluid flow axis of the second connector (11),
wherein the engaged configuration determines an axial constraint between the first and the second connectors (1, 11), in particular said axial constraint being along the fluid flow axis of the first connector (1) and/or of the second connector (11).

3. The connection system of any of the preceding claims, wherein the coupled configuration is defined at least at an end stage of the engagement rotation between the collar (20) and the second connector (11).

4. The connection system of the previous claim 3, wherein the first connector comprises an auxiliary abutment portion (6), said auxiliary abutment portion being configured to block an axial sliding of the collar (20) along a direction opposite to said axial direction, and
wherein the collar (20) is:
- axially constrained to the first connector (1) through said main and auxiliary abutment portions (5, 6) of the first connector (1), so that an axial movement of the collar (20) with respect to the first connector (1) is substantially prevented; or
- axially movable with respect to the first connector within an axial gap defined between said main and auxiliary abutment portions (5, 6) of the first connector (1), and axially constrained to the first connector (1) out of said axial gap, in particular said axial movement being along the fluid flow axis of the first connector (1) and/or of the second connector (11).

5. The connection system of claims 3 or 4, wherein the first connector (1) comprises a main body having tubular shape defining an internal channel (7) for passage of the fluid, said main and auxiliary abutment portions (5, 6) radially extending away from said main body, the first connector (1) further comprising a recess (8) interposed between the main and the auxiliary abutment portions (5, 6),
wherein the abutment portion (25) of the collar (20) is arranged between the main and the auxiliary abutment portions (5, 6) of the first connector (1) into said recess (8),
optionally the collar (20) being axially movable with respect to the first connector (1) of an amount proportional to the distance between the main and the auxiliary abutment portions (5, 6) of the first connector (1).

6. The connection system of any of the preceding claims, wherein the fluid coupling portion (2) of the first connector (1) includes a tip portion (2a) defining an internal channel (7) for fluid transport, in particular said tip portion (2a) having a tapered external surface, wherein the fluid coupling portion (12) of the second connector (11) includes a terminal portion (12a) defining an internal channel (17) for fluid transport, in particular said terminal portion (12a) of the second connector (11) having a tapered internal surface optionally counter shaped to the tapered external surface of the tip portion (2a) of the first connector (1), and
wherein at least part of the tip portion (2a) of the first connector (1) is configured to enter into the terminal portion (12a) of the second connector (11), in particular said a tapered external surface coupling to said tapered internal surface to connect the first and the second connectors (1, 11) in a fluid tight manner.

7. The connection system of any of the preceding claims, wherein the collar (20) comprises a main body having tubular shape extending between:
a first opening (21) configured to receive the second connector (11), in particular to receive the coupling portion (12), the hooking portion (13) and the at least one locking projection (14) of the second connector (11); and
a second opening (22) receiving by insertion the first connector (1), the second opening (22) being arranged at a recess (8) of the first connector (1) to allow coupling, and an abutment portion (25) of the collar (20) extending radially inwardly being arranged at the second opening (22)
the collar surrounding externally at least part of the first connector (1) and the main body of the collar (20) being coaxial with the first connector (1).

8. The connection system of the previous claim 7, wherein:
- the at least one locking projection (24) of the collar (20) is arranged at an inlet of the first opening (21), the hooking portion (23) of the collar (20) being interposed between said at least one locking projection (24) and the second opening (22) of the collar (20); and
- the hooking portion (13) of the second connector (11) is interposed between the at least one locking projection (14) and a terminal portion (12a) of the coupling portion (12) of the second connector (11).

9. The connection system of any of the preceding claims, wherein the collar (20) comprises a main body having tubular shape extending in depth between an internal lateral wall (20a) and an external lateral wall (20b), the collar (20) being mounted over the first connector (1) so that the main body externally surrounds at least part of the first connector (11), in particular surrounding at least part of the coupling portion (2) of the first connector (1),
said hooking portion (23) of the collar (20), in particular said threads of the collar (20), being arranged on said internal lateral wall (20a) of the collar (20),
the at least one locking projection (24) of the collar (20) being radially arranged on the same internal lateral wall (20a) of the collar (20),
in particular said hooking portion (23) of the collar being side by side with said at least one locking projection (24) of the collar (20).

10. The connection system of any of the preceding claims, wherein the second connector (11) has tubular shape defining an internal channel (17) for fluid transport, said tubular shape of the second connector (11) comprising an external lateral wall (11a),
said hooking portion (13) of the second connector (11), in particular said threads of the second connector (11), being arranged on said external lateral wall (11a) of the second connector (11), in particular said hooking portion (13) being arranged at the coupling portion (12) of the second connector (11),
the at least one locking projection (14) of the second connector (11) being radially arranged on said external lateral wall (11a) of the second connector (11), in particular said at least one locking projection (14) being arranged at the coupling portion (12) of the second connector (11),
in particular said hooking portion (13) of the second connector (11) being side by side with said at least one locking projection (14) of the second connector (11).

11. The connection system of any of the preceding claims, wherein both the at least one locking projection (14) of the second connector (11) and the at least one locking projection (24) of the collar (20) comprise respectively a plurality of socket teeth, in particular said socket teeth being wedge-shaped or having a saw tooth shape,
so that when the socket teeth of the collar (20) face, in particular contact, the socket teeth of the second connector (11), the engagement rotation is allowed and the disengagement rotation is prevented.

12. The connection system of the preceding claim 11, wherein the socket teeth of both the second connector (11) and the collar (20) radially define recesses and protrusions alternately,
the second connector (11) defining a maximum external diameter of encumbrance measured at the protrusions of the socket teeth,
the collar (20) having tubular shape and presenting a minimum internal diameter measured at the protrusions of the socket teeth of the collar (20),
said maximum external diameter of encumbrance being higher than said minimum internal diameter so that, when the socket teeth of the second connector (11) are faced to, in particular in contact with, the socket teeth of the collar (20), the protrusions of the socket teeth of the second connector (11) are inserted into the recesses of the socket teeth of the collar (20) and vice versa.

13. The connection of any of the preceding claims, wherein the first connector (1) and the collar (20) are made in two separate pieces subsequently axially coupled, the collar (20) being free to rotate with respect to the first connector (1) at least when the second connector (11) is not coupled to the collar (20) and to the first connector (1) and wherein, when the connection system (100) is disposed in the engaged configuration, at least part of the coupling portion (12) of the second connector (11), is interposed between the collar (20) and at least part of the coupling portion (2) of the first connector (1).

14. The connection system of any of the preceding claims, comprising a fluid line (51), wherein
the first connector (1) extends in length between its coupling portion (2) and a respective connection port (9), said connection port (9) being fixed to an end of said fluid line (51) of a medical machine (200); and/or
the second connector (11) extends in length between its coupling portion (12) and a respective connection port (19), said connection port (19) being fixed to an end of said fluid line (51) of a medical machine (200).

15. Disposable set (50) for a blood treatment apparatus, said disposable comprising:
- a blood circuit (50a) including:
• an access line (52) configured to be connected to a patient (300) and to an inlet port (201a) of a blood treatment unit (201);
• a return line (53) configured to be connected to the patient (300) and to an outlet port (201b) of the blood treatment unit (201);
- optionally an infusion line (54) connected to the blood circuit (52) and/or configured to be connected to the patient (300);
- optionally a monitoring line (55) connected to the blood circuit (50a) or to the infusion line (54);
- at least one connection system (100) according to any of the claims from 1 to 14 connected to at least one between the access line (52), the return line (53), the infusion line (54) and the monitoring line (55).

## Patentansprüche

1. Nichtentfernbares Verbindungssystem (100) für eine medizinische Vorrichtung, wobei das Verbindungssystem (100) Folgendes umfasst:
- einen ersten Verbinder (1), der einen Fluidkopplungsabschnitt (2) umfasst,
- einen zweiten Verbinder (11), der einen jeweiligen Fluidkopplungsabschnitt (12) umfasst, der fluiddicht mit dem Fluidkopplungsabschnitt (2) des ersten Verbinders (1) verbindbar ist, so dass ein Fluid innen zwischen dem ersten und dem zweiten Verbinder (1, 11) fließen kann, wobei der zweite Verbinder (11) ferner einen Hakenabschnitt (13) und mindestens einen Verriegelungsvorsprung (14) umfasst,
- einen Bund (20), der an den ersten Verbinder (1) gekoppelt ist und einen jeweiligen Hakenabschnitt (23) umfasst, der durch eine Eingriffsdrehung entlang einer Eingriffsrichtung (ER) mit dem Hakenabschnitt (13) des zweiten Verbinders (11) in Eingriff bringbar ist, wobei der Hakenabschnitt (23) des Bunds (20) und der Hakenabschnitt (13) des zweiten Verbinders (11) in einer Eingriffskonfiguration das axiale Entfernen des Bunds (20) aus dem zweiten Verbinder (11) verhindern,
wobei sowohl der Hakenabschnitt (23) des Bunds (20) als auch der Hakenabschnitt (13) des zweiten Verbinders Folgendes umfassen:
jeweilige Gewinde, die dazu ausgestaltet sind, einander durch die Eingriffsdrehung in Eingriff zu nehmen, um die Eingriffskonfiguration zu definieren, oder
jeweilige hinterschnittene Gelenke, die dazu ausgestaltet sind, einander durch die Eingriffsdrehung als eine Bajonettkopplung in Eingriff zu nehmen, um die Eingriffskonfiguration zu definieren,
und wobei der erste Verbinder (1) einen Hauptanlageabschnitt (5) umfasst und der Bund (20) einen jeweiligen Anlageabschnitt (25) umfasst, der dazu ausgestaltet ist, den Hauptanlageabschnitt (5) des ersten Verbinders (1) zu kontaktieren, wenn er in einer Anlagekonfiguration angeordnet ist, wobei der Bund (20) in der Anlagekonfiguration bezüglich des ersten Verbinders (1) mindestens entlang einer axialen Richtung axial festgehalten ist, so dass der Bund (20), mindestens wenn das Verbindungssystem (100) in der Eingriffskonfiguration angeordnet ist, den ersten Verbinder (1) mit dem zweiten Verbinder (11) axial in Eingriff bringt,
wobei der Bund (20) ferner einen jeweiligen mindestens einen Verriegelungsvorsprung (24) umfasst, der durch die Eingriffsdrehung des Bunds (20) bezüglich des zweiten Verbinders (11) entlang der Eingriffsrichtung (ER) mit dem mindestens einen Verriegelungsvorsprung (14) des zweiten Verbinders (11) in Eingriff bringbar ist,
wobei das Verbindungssystem (100) mittels der Eingriffsdrehung in mindestens einem entriegelten Zustand ausgestaltbar ist, wobei der Bund (20) durch Drehung bezüglich des ersten Verbinders (1) beweglich ist und der mindestens eine Verriegelungsvorsprung (24) des Bunds (20) und der mindestens eine Verriegelungsvorsprung (14) des zweiten Verbinders (11) in einer entkoppelten Konfiguration sind, **dadurch gekennzeichnet, dass** der mindestens eine Verriegelungsvorsprung (24) des Bunds (20) und der mindestens eine Verriegelungsvorsprung (14) des zweiten Verbinders (11) in einer gekoppelten Konfiguration eine Ausdrückdrehung des Bunds (20) bezüglich des zweiten Verbinders (11) verhindern, wobei es sich bei der Ausrückdrehung um eine Drehung in die entgegengesetzte Richtung bezüglich der Eingriffsdrehung handelt,
wobei das Verbindungssystem (100) aus dem entriegelten Zustand in einen verriegelten Zustand ausgestaltbar ist, wobei der mindestens eine Verriegelungsvorsprung (24) des Bunds (20) und der mindestens eine Verriegelungsvorsprung (14) des zweiten Verbinders (11) in der gekoppelten Konfiguration sind und der Hakenabschnitt (23) des Bunds (20) und der Hakenabschnitt (13) des zweiten Verbinders (11) in der Eingriffskonfiguration sind, wobei der Bund (20) in dem verriegelten Zustand durch die Eingriffsdrehung nicht entfernbar mit dem zweiten Verbinder (11) in Eingriff steht und der mindestens eine Verriegelungsvorsprung (14) und der Hakenabschnitt (13) des zweiten Verbinders (11) jeweils mit dem mindestens einen Verriegelungsvorsprung (24) und dem Hakenabschnitt (23) des Bunds (20) zusammenwirken, um ein Ausrücken des ersten Verbinders (1) aus dem zweiten Verbinder (11) zu verhindern.

2. Verbindungssystem nach Anspruch 1, wobei:
sich der erste Verbinder (1), insbesondere der Kopplungsabschnitt (2) des ersten Verbinders (1), längenmäßig entlang einer Fluidströmungsachse erstreckt,
sich der zweite Verbinder (11), insbesondere der Kopplungsabschnitt (12) des zweiten Verbinders (11), längenmäßig entlang einer jeweiligen Fluidströmungsachse erstreckt,
wobei die Fluidströmungsachse des ersten Verbinders (1) mindestens dann, wenn der Kopplungsabschnitt (2) des ersten Verbinders (1) mit dem Kopplungsabschnitt (12) des zweiten Verbinders (11) verbunden ist, auf die Fluidströmungsachse des zweiten Verbinders (11) ausgerichtet ist,
wobei die Eingriffskonfiguration ein axiales Festhalten zwischen dem ersten und dem zweiten Verbinder (1, 11) bestimmt, wobei das axiale Festhalten insbesondere entlang der Fluidströmungsachse des ersten Verbinders (1) und/oder des zweiten Verbinders (11) vorliegt.

3. Verbindungssystem nach einem der vorhergehenden Ansprüche, wobei die gekoppelte Konfiguration mindestens in einem Endstadium der Eingriffsdrehung zwischen dem Bund (20) und dem zweiten Verbinder (11) definiert ist.

4. Verbindungssystem nach dem vorhergehenden Anspruch 3, wobei der erste Verbinder einen Hilfsanlageabschnitt (6) umfasst, wobei der Hilfsanlageabschnitt dazu ausgestaltet ist, ein axiales Gleiten des Bunds (20) entlang einer der axialen Richtung entgegengesetzt verlaufenden Richtung zu blockieren, und wobei der Bund (20)
- durch den Haupt- und den Hilfsanlageabschnitt (5, 6) des ersten Verbinders (1) axial an dem ersten Verbinder (1) festgehalten ist, so dass eine axiale Bewegung des Bunds (20) bezüglich des ersten Verbinders (1) im Wesentlichen verhindert wird, oder
- bezüglich des ersten Verbinders in einem zwischen dem Haupt- und dem Hilfsanlageabschnitt (5, 6) des ersten Verbinders (1) definierten axialen Spalt axial beweglich und außerhalb des axialen Spalts axial an dem ersten Verbinder (1) festgehalten ist, wobei die axiale Bewegung insbesondere entlang der Fluidströmungsachse des ersten Verbinders (1) und/oder des zweiten Verbinders (11) erfolgt.

5. Verbindungssystem nach Ansprüchen 3 oder 4, wobei der erste Verbinder (1) einen Hauptkörper umfasst, der rohrförmig ist und einen inneren Kanal (7) für den Durchgang des Fluids definiert, wobei sich der Haupt- und der Hilfsanlageabschnitt (5, 6) radial von dem Hauptkörper weg erstrecken, wobei der erste Verbinder (1) ferner eine Aussparung (8) umfasst, die zwischen dem Haupt- und dem Hilfsanlageabschnitt (5, 6) angeordnet ist,
wobei der Anlageabschnitt (25) des Bunds (20) zwischen dem Haupt- und dem Hilfsanlageabschnitt (5, 6) des ersten Verbinders (1) in der Aussparung (8) angeordnet ist,
optional wobei der Bund (20) axial bezüglich des ersten Verbinders (1) in einem Ausmaß beweglich ist, das zu dem Abstand zwischen dem Haupt- und dem Hilfsanlageabschnitt (5, 6) des ersten Verbinders (1) proportional ist.

6. Verbindungssystem nach einem der vorhergehenden Ansprüche, wobei der Fluidkopplungsabschnitt (2) des ersten Verbinders (1) einen Spitzenabschnitt (2a) aufweist, der einen inneren Kanal (7) für den Fluidtransport definiert, insbesondere wobei der Spitzenabschnitt (2a) eine sich verjüngende Außenfläche hat,
wobei der Fluidkopplungsabschnitt (12) des zweiten Verbinders (11) einen Endabschnitt (12a) aufweist, der einen inneren Kanal (17) für den Fluidtransport definiert, insbesondere wobei der Endabschnitt (12a) des zweiten Verbinders (11) eine sich verjüngende Innenfläche hat, die optional die entsprechende Gegenform der sich verjüngenden Außenfläche des Spitzenabschnitts (2a) des ersten Verbinders (1) hat, und
wobei mindestens ein Teil des Spitzenabschnitts (2a) des ersten Verbinders (1) dazu ausgestaltet ist, in den Endabschnitt (12a) des zweiten Verbinders (11) einzutreten, insbesondere wobei die sich verjüngende Außenfläche mit der sich verjüngenden Innenfläche koppelt, um den ersten und den zweiten Verbinder (1, 11) fluiddicht miteinander zu verbinden.

7. Verbindungssystem nach einem der vorhergehenden Ansprüche, wobei der Bund (20) einen rohrförmigen Hauptkörper umfasst, der sich zwischen:
einer ersten Öffnung (21), die dazu ausgestaltet ist, den zweiten Verbinder (11) aufzunehmen und insbesondere den Kopplungsabschnitt (12), den Hakenabschnitt (13) und den mindestens einen Verriegelungsvorsprung (14) des zweiten Verbinders (11) aufzunehmen, und
einer zweiten Öffnung (22) erstreckt, die den ersten Verbinder (1) durch Einführen aufnimmt, wobei die zweite Öffnung (22) an einer Aussparung (8) des ersten Verbinders (1) angeordnet ist, um die Koppelung zu gestatten, und wobei ein sich radial nach innen erstreckender Anlageabschnitt (25) des Bunds (20) an der zweiten Öffnung (22) angeordnet ist, wobei der Bund mindestens einen Teil des ersten Verbinders (1) außen umgibt und der Hauptkörper des Bunds (20) mit dem ersten Verbinder (1) koaxial ist.

8. Verbindungssystem nach dem vorhergehenden Anspruch 7, wobei:
- der mindestens eine Verriegelungsvorsprung (24) des Bunds (20) an einem Einlass der ersten Öffnung (21) angeordnet ist, wobei der Hakenabschnitt (23) des Bunds (20) zwischen dem mindestens einen Verriegelungsvorsprung (24) und der zweiten Öffnung (22) des Bunds (20) angeordnet ist, und
- der Hakenabschnitt (13) des zweiten Verbinders (11) zwischen dem mindestens einen Verriegelungsvorsprung (14) und einem Endabschnitt (12a) des Kopplungsabschnitts (12) des zweiten Verbinders (11) angeordnet ist.

9. Verbindungssystem nach einem der vorhergehenden Ansprüche, wobei der Bund (20) einen rohrförmigen Hauptkörper umfasst, der sich tiefenmäßig zwischen einer inneren Seitenwand (20a) und einer äußeren Seitenwand (20b) erstreckt, wobei der Bund (20) über dem ersten Verbinder (1) montiert ist, so dass der Hauptkörper mindestens einen Teil des ersten Verbinders (11) außen umgibt, insbesondere mindestens einen Teil des Kopplungsabschnitts (2) des ersten Verbinders (1) umgibt,
wobei der Hakenabschnitt (23) des Bunds (20), insbesondere das Gewinde des Bunds (20), an der inneren Seitenwand (20a) des Bunds (20) angeordnet ist, wobei der mindestens eine Verriegelungsvorsprung (24) des Bunds (20) radial an derselben inneren Seitenwand (20a) des Bunds (20) angeordnet ist, insbesondere wobei der Hakenabschnitt (23) des Bunds und der mindestens eine Verriegelungsvorsprung (24) des Bunds (20) nebeneinander angeordnet sind.

10. Verbindungssystem nach einem der vorhergehenden Ansprüche, wobei der zweite Verbinder (11) rohrförmig ist und einen inneren Kanal (17) für den Fluidtransport definiert, wobei die Rohrform des zweiten Verbinders (11) eine äußere Seitenwand (11a) umfasst,
wobei der Hakenabschnitt (13) des zweiten Verbinders (11), insbesondere das Gewinde des zweiten Verbinders (11), an der äußeren Seitenwand (11a) des zweiten Verbinders (11) angeordnet ist, insbesondere wobei der Hakenabschnitt (13) an dem Kopplungsabschnitt (12) des zweiten Verbinders (11) angeordnet ist,
wobei der mindestens eine Verriegelungsvorsprung (14) des zweiten Verbinders (11) radial an der äußeren Seitenwand (11a) des zweiten Verbinders (11) angeordnet ist, insbesondere wobei mindestens ein Verriegelungsvorsprung (14) an dem Kopplungsabschnitt (12) des zweiten Verbinders (11) angeordnet ist,
insbesondere wobei der Hakenabschnitt (13) des zweiten Verbinders (11) und der mindestens eine Verriegelungsvorsprung (14) des zweiten Verbinders (11) nebeneinander angeordnet sind.

11. Verbindungssystem nach einem der vorhergehenden Ansprüche, wobei sowohl der mindestens eine Verriegelungsvorsprung (14) des zweiten Verbinders (11) als auch der mindestens eine Verriegelungsvorsprung (24) des Bunds (20) jeweils eine Vielzahl von Buchsenzähnen umfassen, insbesondere wobei die Buchsenzähne keilförmig sind oder sägezahnförmig sind,
so dass wenn die Buchsenzähne des Bunds (20) den Buchsenzähnen des zweiten Verbinders (11) zugewandt sind, insbesondere diese kontaktieren, die Eingriffsdrehung gestattet ist und die Ausrückdrehung verhindert wird.

12. Verbindungssystem nach dem vorhergehenden Anspruch 11, wobei die Buchsenzähne sowohl des zweiten Verbinders (11) als auch des Bunds (20) radial abwechselnd Aussparungen und Vorsprünge definieren, wobei der zweite Verbinder (11) einen maximalen äußeren Behinderungsdurchmesser definiert, der an den Vorsprüngen der Buchsenzähne gemessen wird,
wobei der Bund (20) rohrförmig ist und einen minimalen inneren Durchmesser aufweist, der an den Vorsprüngen der Buchsenzähne des Bunds (20) gemessen wird,
wobei der maximale äußere Behinderungsdurchmesser höher als der minimale innere Durchmesser ist, so dass die Vorsprünge der Buchsenzähne des zweiten Verbinders (11) in die Aussparungen der Buchsenzähne des Bunds (20) eingeführt werden und umgekehrt, wenn die Buchsenzähne des zweiten Verbinders (11) den Buchsenzähnen des Bunds (20) zugewandt sind, insbesondere diese kontaktieren.

13. Verbindungssystem nach einem der vorhergehenden Ansprüche, wobei der erste Verbinder (1) und der Bund (20) aus zwei separaten Teilen hergestellt sind, die anschließend axial gekoppelt werden, wobei sich der Bund (20) frei bezüglich des ersten Verbinders (1) drehen kann, zumindest wenn der zweite Verbinder (11) nicht an den Bund (20) und an den ersten Verbinder (1) gekoppelt ist, und wobei mindestens ein Teil des Kopplungsabschnitts (12) des zweiten Verbinders (11) zwischen dem Bund (20) und mindestens einem Teil des Kopplungsabschnitts (2) des ersten Verbinders (1) angeordnet ist, wenn das Verbindungssystem (100) in der Eingriffskonfiguration angeordnet ist.

14. Verbindungssystem nach einem der vorhergehenden Ansprüche, umfassend eine Fluidleitung (51), wobei sich der erste Verbinder (1) längenmäßig zwischen seinem Kopplungsabschnitt (2) und einem jeweiligen Verbindungsport (9) erstreckt, wobei der Verbindungsport (9) an einem Ende der Fluidleitung (51) einer medizinischen Maschine (200) befestigt ist und/oder
sich der zweite Verbinder (11) längenmäßig zwischen seinem Kopplungsabschnitt (12) und einem jeweiligen Verbindungsport (19) erstreckt, wobei der Verbindungsport (19) an einem Ende der Fluidleitung (51) einer medizinischen Maschine (200) befestigt ist.

15. Einwegsatz (50) für eine Blutbehandlungsvorrichtung, wobei der Einwegsatz Folgendes umfasst:
- einen Blutkreislauf (50a), aufweisend:
• eine Zuflussleitung (52), die dazu ausgestaltet ist, mit einem Patienten (300) und mit einem Einlass-Port (201a) einer Blutbehandlungseinheit (201) verbunden zu werden,
• eine Rückflussleitung (53), die dazu ausgestaltet ist, mit einem Patienten (300) und mit einem Auslass-Port (201b) der Blutbehandlungseinheit (201) verbunden zu werden,
- optional eine Infusionsleitung (54), die mit dem Blutkreislauf (52) verbunden und/oder dazu ausgestaltet ist, mit dem Patienten (300) verbunden zu werden,
- optional eine Überwachungsleitung (55), die mit dem Blutkreislauf (50a) oder mit der Infusionsleitung (54) verbunden ist,
- mindestens ein Verbindungssystem (100) nach einem der Ansprüche 1 bis 14, das mit der Zuflussleitung (52) und/oder der Rückflussleitung (53) und/oder der Infusionsleitung (54) und/oder der Überwachungsleitung (55) verbunden ist.

## Revendications

1. Système de connexion non amovible (100) pour un appareil médical, ledit système de connexion (100) comprenant :
- un premier connecteur (1) comprenant une partie de couplage de fluide (2) ;
- un second connecteur (11) comprenant une partie de couplage de fluide respective (12) pouvant être connectée, de manière étanche aux fluides, à la partie de couplage de fluide (2) du premier connecteur (1), de sorte qu'un fluide est capable de s'écouler de manière interne entre le premier et le second connecteur (1, 11), le second connecteur (11) comprenant en outre une partie d'accrochage (13) et au moins une saillie de verrouillage (14) ;
- un collier (20) couplé au premier connecteur (1) et comprenant une partie d'accrochage respective (23) pouvant s'engager dans la partie d'accrochage (13) du second connecteur (11) par l'intermédiaire d'une rotation d'engagement le long d'une direction d'engagement (ER), dans une configuration engagée, la partie d'accrochage (23) du collier (20) et la partie d'accrochage (13) du second connecteur (11) empêchant le retrait axial du collier (20) du second connecteur (11), la partie d'accrochage (23) du collier (20) et la partie d'accrochage (13) du second connecteur comprenant toutes deux :
des filetages respectifs configurés pour s'engager les uns avec les autres par l'intermédiaire de la rotation d'engagement pour définir la configuration engagée ; ou
des joints en contre-dépouille respectifs configurés pour s'engager les uns avec les autres comme un couplage à baïonnette par l'intermédiaire de la rotation d'engagement pour définir la configuration engagée ;
et le premier connecteur (1) comprenant une partie de butée principale (5) et le collier (20) comprenant une partie de butée respective (25) configurée pour entrer en contact avec la partie de butée principale (5) du premier connecteur (1) lorsqu'il est disposé dans une configuration de butée, dans ladite configuration de butée, le collier (20) étant contraint axialement par rapport au premier connecteur (1) au moins le long d'une direction axiale, de sorte que, au moins lorsque le système de connexion (100) est disposé dans la configuration engagée, le collier (20) engage axialement le premier connecteur (1) avec le second connecteur (11),
le collier (20) comprenant en outre au moins une saillie de verrouillage respective (24) pouvant s'engager avec l'au moins une saillie de verrouillage (14) du second connecteur (11) par l'intermédiaire de la rotation d'engagement du collier (20) par rapport au second connecteur (11) le long de la direction d'engagement (ER) ;
au moyen de la rotation d'engagement, le système de connexion (100) pouvant être configuré dans au moins un état déverrouillé, le collier (20) étant mobile par rotation par rapport au premier connecteur (1) et l'au moins une saillie de verrouillage (24) du collier (20) et l'au moins une saillie de verrouillage (14) du second connecteur (11) étant dans une configuration découplée ;
**caractérisé par le fait que**, dans une configuration couplée, l'au moins une saillie de verrouillage (24) du collier (20) et l'au moins une saillie de verrouillage (14) du second connecteur (11) empêchent une rotation de désengagement du collier (20) par rapport au second connecteur (11), ladite rotation de désengagement étant une rotation dans une direction opposée par rapport à ladite rotation d'engagement,
le système de connexion (100) étant configurable de l'état déverrouillé à un état verrouillé, l'au moins une saillie de verrouillage (24) du collier (20) et l'au moins une saillie de verrouillage (14) du second connecteur (11) étant dans la configuration couplée, et la partie d'accrochage (23) du collier (20) et la partie d'accrochage (13) du second connecteur (11) étant dans la configuration engagée, dans ledit état verrouillé, le collier (20) étant engagé de manière inamovible sur le second connecteur (11) par l'intermédiaire de ladite rotation d'engagement et l'au moins une saillie de verrouillage (14) et la partie d'accrochage (13) du second connecteur (11) coopérant respectivement avec l'au moins une saillie de verrouillage (24) et la partie d'accrochage (23) du collier (20) empêchant le désengagement du premier connecteur (1) du second connecteur (11).

2. Système de connexion selon la revendication 1,
le premier connecteur (1), en particulier la partie de couplage (2) du premier connecteur (1), s'étendant en longueur le long d'un axe d'écoulement de fluide ;
le second connecteur (11), en particulier la partie de couplage (12) du second connecteur (11), s'étendant en longueur le long d'un axe d'écoulement de fluide respectif ;
l'axe d'écoulement de fluide du premier connecteur (1) étant aligné, au moins lorsque la partie de couplage (2) du premier connecteur (1) est connectée à la partie de couplage (12) du second connecteur (11), avec l'axe d'écoulement de fluide du second connecteur (11),
la configuration engagée déterminant une contrainte axiale entre les premier et second connecteurs (1, 11), en particulier ladite contrainte axiale étant le long de l'axe d'écoulement de fluide du premier connecteur (1) et/ou du second connecteur (11).

3. Système de connexion selon l'une quelconque des revendications précédentes, la configuration couplée étant définie au moins à un stade final de la rotation d'engagement entre le collier (20) et le second connecteur (11).

4. Système de connexion selon la revendication précédente 3, le premier connecteur comprenant une partie de butée auxiliaire (6), ladite partie de butée auxiliaire étant configurée pour bloquer un coulissement axial du collier (20) le long d'une direction opposée à ladite direction axiale, et
le collier (20) étant :
- contraint axialement par rapport au premier connecteur (1) par l'intermédiaire desdites parties de butée principale et auxiliaire (5, 6) du premier connecteur (1), de sorte qu'un mouvement axial du collier (20) par rapport au premier connecteur (1) est sensiblement empêché ; ou
- mobile axialement par rapport au premier connecteur à l'intérieur d'un espace axial défini entre lesdites parties de butée principale et auxiliaire (5, 6) du premier connecteur (1), et contraint axialement par rapport au premier connecteur (1) en dehors dudit espace axial, en particulier ledit mouvement axial étant le long de l'axe d'écoulement de fluide du premier connecteur (1) et/ou du second connecteur (11).

5. Système de connexion selon la revendication 3 ou 4, le premier connecteur (1) comprenant un corps principal ayant une forme tubulaire définissant un canal interne (7) pour le passage du fluide, lesdites parties de butée principale et auxiliaire (5, 6) s'étendant radialement à l'écart dudit corps principal, le premier connecteur (1) comprenant en outre un évidement (8) interposé entre les parties de butée principale et auxiliaire (5, 6),
la partie de butée (25) du collier (20) étant agencée entre les parties de butée principale et auxiliaire (5, 6) du premier connecteur (1) dans ledit évidement (8),
le collier (20) étant éventuellement mobile axialement par rapport au premier connecteur (1) d'une quantité proportionnelle à la distance entre les parties de butée principale et auxiliaire (5, 6) du premier connecteur (1).

6. Système de connexion selon l'une quelconque des revendications précédentes, la partie de couplage de fluide (2) du premier connecteur (1) comprenant une partie de pointe (2a) définissant un canal interne (7) pour le transport de fluide, en particulier ladite partie de pointe (2a) ayant une surface externe effilée,
la partie de couplage de fluide (12) du second connecteur (11) comprenant une partie terminale (12a) définissant un canal interne (17) pour le transport de fluide, en particulier ladite partie terminale (12a) du second connecteur (11) ayant une surface interne effilée éventuellement de forme contraire à la surface externe effilée de la partie de pointe (2a) du premier connecteur (1), et
au moins une partie de la partie de pointe (2a) du premier connecteur (1) étant configurée pour entrer dans la partie terminale (12a) du second connecteur (11), en particulier ladite surface externe effilée se couplant à ladite surface interne effilée pour connecter les premier et second connecteurs (1, 11) d'une manière étanche aux fluides.

7. Système de connexion selon l'une quelconque des revendications précédentes, le collier (20) comprenant un corps principal de forme tubulaire s'étendant entre :
une première ouverture (21) configurée pour recevoir le second connecteur (11), en particulier pour recevoir la partie de couplage (12), la portion d'accrochage (13) et l'au moins une saillie de verrouillage (14) du second connecteur (11) ; et
une seconde ouverture (22) recevant par insertion le premier connecteur (1), la seconde ouverture (22) étant agencée au niveau d'un évidement (8) du premier connecteur (1) pour permettre le couplage, et une partie de butée (25) du collier (20) s'étendant radialement vers l'intérieur étant agencée au niveau de la seconde ouverture (22),
le collier entourant extérieurement au moins une partie du premier connecteur (1) et le corps principal du collier (20) étant coaxial avec le premier connecteur (1).

8. Système de connexion selon la revendication précédente 7,
- l'au moins une saillie de verrouillage (24) du collier (20) étant agencée au niveau d'une entrée de la première ouverture (21), la partie d'accrochage (23) du collier (20) étant interposée entre ladite au moins une saillie de verrouillage (24) et la seconde ouverture (22) du collier (20) ; et
- la partie d'accrochage (13) du second connecteur (11) étant interposée entre l'au moins une saillie de verrouillage (14) et une partie terminale (12a) de la partie de couplage (12) du second connecteur (11).

9. Système de connexion selon l'une quelconque des revendications précédentes, le collier (20) comprenant un corps principal de forme tubulaire s'étendant en profondeur entre une paroi latérale interne (20a) et une paroi latérale externe (20b), le collier (20) étant monté sur le premier connecteur (1) de sorte que le corps principal entoure extérieurement au moins une partie du premier connecteur (11), en particulier entourant au moins une partie de la partie de couplage (2) du premier connecteur (1),
ladite partie d'accrochage (23) du collier (20), en particulier lesdits filets du collier (20), étant agencée sur ladite paroi latérale interne (20a) du collier (20), l'au moins une saillie de verrouillage (24) du collier (20) étant agencée radialement sur la même paroi latérale interne (20a) du collier (20),
en particulier ladite partie d'accrochage (23) du collier étant côte à côte avec ladite au moins une saillie de verrouillage (24) du collier (20).

10. Système de connexion selon l'une quelconque des revendications précédentes, le second connecteur (11) ayant une forme tubulaire définissant un canal interne (17) pour le transport de fluide, ladite forme tubulaire du second connecteur (11) comprenant une paroi latérale externe (11a),
ladite partie d'accrochage (13) du second connecteur (11), en particulier lesdits filets du second connecteur (11), étant agencée sur ladite paroi latérale externe (11a) du second connecteur (11), en particulier ladite partie d'accrochage (13) étant agencée au niveau de la partie de couplage (12) du second connecteur (11),
l'au moins une saillie de verrouillage (14) du second connecteur (11) étant agencée radialement sur ladite paroi latérale externe (11a) du second connecteur (11), en particulier ladite au moins une saillie de verrouillage (14) étant agencée au niveau de la partie de couplage (12) du second connecteur (11),
en particulier ladite partie d'accrochage (13) du second connecteur (11) étant côte à côte avec ladite au moins une saillie de verrouillage (14) du second connecteur (11).

11. Système de connexion selon l'une quelconque des revendications précédentes, à la fois l'au moins une saillie de verrouillage (14) du second connecteur (11) et l'au moins une saillie de verrouillage (24) du collier (20) comprenant respectivement une pluralité de dents d'emboîtement, en particulier lesdites dents d'emboîtement étant en forme de coin ou ayant une forme de dent de scie, de sorte que lorsque les dents d'emboîtement du collier (20) sont en regard, en particulier sont en contact, avec les dents d'emboîtement du second connecteur (11), la rotation d'engagement est permise et la rotation de désengagement est empêchée.

12. Système de connexion selon la revendication précédente 11, les dents d'emboîtement du second connecteur (11) et du collier (20) définissant radialement des évidements et des saillies en alternance,
le second connecteur (11) définissant un diamètre extérieur maximal d'encombrement mesuré au niveau des saillies des dents d'emboîtement,
le collier (20) ayant une forme tubulaire et présentant un diamètre interne minimum mesuré au niveau des saillies des dents d'emboîtement du collier (20),
ledit diamètre extérieur maximal d'encombrement étant supérieur audit diamètre intérieur minimal de sorte que, lorsque les dents d'emboîtement du second connecteur (11) sont en regard, en particulier en contact, avec les dents d'emboîtement du collier (20), les saillies des dents d'emboîtement du second connecteur (11) sont insérées dans les évidements des dents d'emboîtement du collier (20) et vice versa.

13. Connexion selon l'une quelconque des revendications précédentes, le premier connecteur (1) et le collier (20) étant réalisés en deux pièces distinctes ultérieurement couplées axialement, le collier (20) étant libre de tourner par rapport au premier connecteur (1) au moins lorsque le second connecteur (11) n'est pas couplé au collier (20) et au premier connecteur (1) et, lorsque le système de connexion (100) est disposé dans la configuration engagée, au moins une partie de la partie de couplage (12) du second connecteur (11) étant interposée entre le collier (20) et au moins une partie de la partie de couplage (2) du premier connecteur (1).

14. Système de connexion selon l'une quelconque des revendications précédentes, comprenant une ligne de fluide (51),
le premier connecteur (1) s'étendant en longueur entre sa partie de couplage (2) et un orifice de connexion respectif (9), ledit orifice de connexion (9) étant fixé à une extrémité de ladite ligne de fluide (51) d'une machine médicale (200) ; et/ou
le second connecteur (11) s'étendant en longueur entre sa partie de couplage (12) et un orifice de connexion respectif (19), ledit orifice de connexion (19) étant fixé à une extrémité de ladite ligne de fluide (51) d'une machine médicale (200).

15. Ensemble jetable (50) pour un appareil de traitement du sang, ledit ensemble jetable comprenant :
- un circuit sanguin (50a) comprenant :
• une ligne d'accès (52) configurée pour être connectée à un patient (300) et à un orifice d'entrée (201a) d'une unité de traitement du sang (201) ;
• une ligne de retour (53) configurée pour être connectée au patient (300) et à un orifice de sortie (201b) de l'unité de traitement du sang (201) ;
- éventuellement une ligne de perfusion (54) connectée au circuit sanguin (52) et/ou configurée pour être connectée au patient (300) ;
- éventuellement une ligne de surveillance (55) connectée au circuit sanguin (50a) ou à la ligne de perfusion (54) ;
- au moins un système de connexion (100) selon l'une quelconque des revendications de 1 à 14 connecté à au moins l'une entre la ligne d'accès (52), la ligne de retour (53), la ligne de perfusion (54) et la ligne de surveillance (55).
